# EUROPEAN PATENT APPLICATION

(11) **EP 2 169 572 A2**
(43) Date of publication of application: **31.03.2010**
(21) Application number: 09170997.2
(22) Date of filing: 22.09.2009
(51) Int. Cl.: G06F 19/00

(54) **System and method for using classification trees to predict rare events**

(30) Priority: 25.09.2008 US 284943
(71) Applicant: AIR PRODUCTS AND CHEMICALS, INC., Allentown, PA 18195-1501 (US)
(72) Inventor: Magent, Michael Andrew, Allentown PA 18104 (US); Neogi, Debashis, Emmaus PA 18049 (US); Mehta, Sanjay, Alburtis PA 18011 (US); Jenkins, Jean, Chicago IL 60615 (US); Waring, Malcolm Meritt, Shawnee on Delaware PA 18356 (US); Lewis, Charles Roland, Fogelsville PA 18051 (US); Toth, Michael S., Allentown PA 18103 (US); Glick, Gregory Robert, Allentown PA 18104 (US); Barbieri, Robert S., Schnecksville PA 18071 (US); Petit, Cecilia Anna Paulette, Quakertown PA 18951 (US)
(74) Representative: Stones, James Alexander

(57) **Abstract**

Systems and methods are provided for predicting rare events, such as hospitalization events. A set of data records, each containing multiple attributes with one or more values (which may include an "unknown" value), may represent a root node of a decision tree. This root node may be partitioned based on one of the attributes, such that the concentration (e.g., "purity") of a relevant outcome (e.g., the rare event) is increased in one node and decreased in another. This process may be repeated until a decision tree with sufficiently pure leaf nodes is created. This "purified" decision tree may then be used to predict one or more rare events.

## Description

### BACKGROUND OF THE INVENTION

Predicting rare events is difficult to model using traditional techniques. Most traditional techniques require balanced datasets to produce an accurate model. In other words, the model construction technique requires approximately equal numbers of target events and non-target events. This is a problem for trying to predict rare events, where the target event does not occur as often as the non-target events. Additionally, traditional techniques can be complicated and unintuitive, making adjustment and experimentation difficult. Traditional techniques often have heavy "pre-processing" costs that slow experimentation down, and generally reduce the ability to produce an accurate model due to time costs.

### BRIEF SUMMARY OF THE INVENTION

Example embodiments of the present invention relate to predicting rare event outcomes using classification trees. One example of a rare event that may be predicted by example embodiments of the present invention is a hospitalization event within a certain time period for a particular person. Hospitalization events are traumatic and expensive, requiring accurate predictions for the benefit of both the patient and insurance companies who insure the patient. Example embodiments of the present invention may create classification trees that essentially comprise a set of rules related to predictor variables. This approach has several advantages over other approaches (e.g., neural networks, regression analysis, etc.). Since the classification trees are essentially a set of structured rules, they can be checked manually for consistency, can be readily and visually explained, and can be readily integrated with other rules. Other approaches create a "black box" situation, where data goes in and a prediction comes out. The logic inside the box is complicated and unintuitive, which does not create a very user-friendly modeling system.

The classification tree may include a root node representing all of the available data records. The data records may then be divided into child nodes that include subsets of the records associated with the parent node. The child nodes may be organized based on one or more attributes of the data records (e.g., age over 30, gender, height, etc.). The goal in the construction of the child nodes may be to increase the concentration of positive outcomes with respect to the relevant event (e.g., hospitalization events) in one child node, and increase the concentration of negative outcomes with respect to the relevant event (e.g., no hospitalization event) in the other child node. Once the tree has achieved a sufficient level of purity in the leaf nodes, the tree may be used to create a model capable of predicting the occurrence of a rare event and an associated confidence of prediction.

### BRIEF DESCRIPTION OF SEVERAL VIEWS OF THE DRAWINGS

Fig. 1A illustrates an example procedure, according to an example embodiment of the present invention.
Fig. 1B illustrates another example procedure, according to an example embodiment of the present invention.
Fig. 2 illustrates an example decision tree, according to an example embodiment of the present invention.
Fig. 3 illustrates an example procedure for constructing a decision tree, according to an example embodiment of the present invention.
Fig. 4 illustrates an example system, according to an example embodiment of the present invention.

### DETAILED DESCRIPTION OF THE INVENTION

Example embodiments of the present invention relate to predicting rare event outcomes using classification trees. One example of a rare event that may be predicted by example embodiments of the present invention is a hospitalization event within a certain time period for a particular person. Hospitalization events are traumatic and expensive, requiring accurate predictions for the benefit of both the patient and insurance companies who insure the patient. Example embodiments of the present invention may create classification trees that essentially comprise a set of rules related to predictor variables. This approach has several advantages over other approaches (e.g., neural networks, regression analysis, etc.). Since the classification trees are essentially a set of structured rules, they can be checked manually for consistency, can be readily and visually explained, and can be readily integrated with other rules.

Decision trees are easily understood, providing a graphical representation of the intuitive logic behind the set of rules those trees represent. In addition, decision trees are very flexible and can handle large datasets with minimal pre-processing of the data. Because of these two benefits, example embodiments of the present invention are easily manipulated to test different modeling situations. Fast, easy, and flexible model adjustments allow for a more accurate predictive model to be refined through adjustment and experimentation.

Data used in the predictor model may be pulled from a number of sources, and the types of data will depend on the event to be predicted. One example may be hospitalization events; meaning, based on data and the sequence of events occurring with respect to a specific person, predicting the likelihood that that person will require hospitalization in any given timeframe. In the example of predicting hospitalization events, relevant data may include: personal data about the patient's background and health data about the patient's medical history, etc. Examples may include: date of birth, height (after a certain age), ethnicity, gender, family history, geography (e.g., place where the patient lives), family size including marital status, career field, education level, medical charts, medical records, medical device data, lab data, weight gain/loss, prescription claims, insurance claims, physical activity levels, climate changes of patient-location, and any number of other medical or health related metrics, or any number of other pieces of data. Data may be pulled from any number of sources, include patient questionnaires, text records (e.g., text data mining of narrative records), data storage of medial devices (e.g., data collected by a heart monitor), health databases, insurance claim databases, etc.

Data that is useful to the model in a native format may be directly imported into a prediction event database. Other data may need to be transformed into a useful state. Still other data may be stored with unnecessary components (e.g., data contained in a text narrative). In this latter situation, a text mining procedure may need to be implemented. Text mining and data mining are known in the art and several commercial products exist for this purpose. However, the use of text mining to populate databases for use in a subsequent data mining or analytical model is not widespread. Alternatively, a proprietary procedure may be used to mine text for relevant event data. Data may be pulled from a number of sources and stored in a central modeling database. The modeling database may consist of one data repository in one location, more than one data repository in one location, or more than one data repository in more than one location. One benefit of example embodiments of the present invention is the flexibility with regard to input data. The decision trees may not require much, if any, data transformation for most data input or imported into the model when compared with other techniques. However, example embodiments may need to have non-events characterized as an event for the decision tree. For example, a single event may be a hospitalization event occurring one month ago. However, if no other hospitalization events occurred then that too is a relevant event that needs to be addressed, i.e., "no hospitalization events in the past month". In this way, so-called "lag" variables may be accounted for, and the event at a specific time and the lack of an event over a specific period may both factor into the decision tree model.

Once the data is stored in the modeling database, different "views" may be created to facilitate different modeling approaches. A view may be created based on any number of characteristics, or combination of characteristics. One simple example may include the time frame of the predicted event. For example, the same set of data may have a modeling view set to predict the probability of a hospitalization event in the next week or the probability of a hospitalization event in the next month.

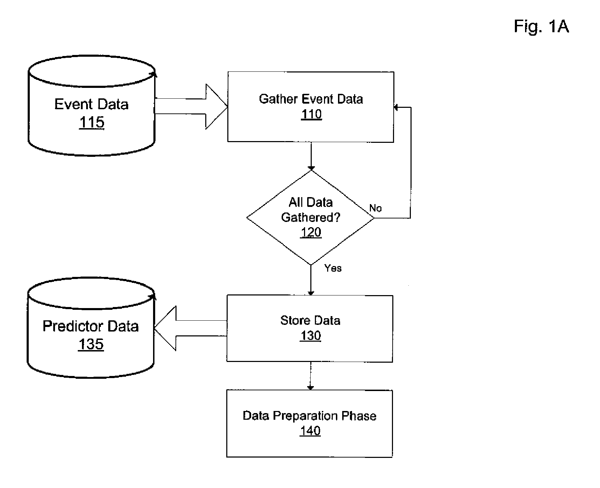

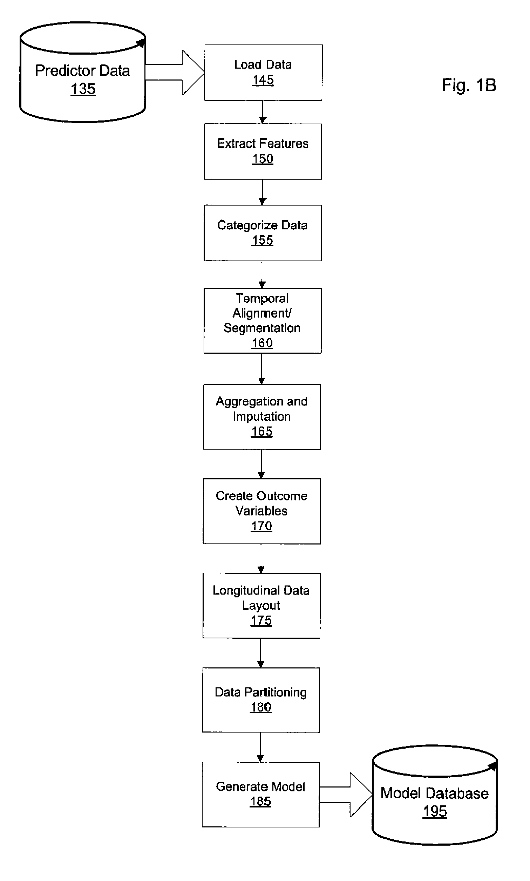

Fig. 1A and Fig. 1B illustrate one example procedure for preparing modeling data, according to an example embodiment of the present invention. The example procedure illustrated in Fig. 1A and Fig. 1B will be discussed in terms of the patient/hospitalization example, but the example procedure could be applied to any event-based prediction model. At 110, the example procedure may gather event data. This could be any kind of data (e.g., the types of data listed above) and could be from any source. Some data may come from the patients themselves. Some data may come from devices associated with patients (e.g., a pacemaker, systems monitor, cellular telephone, etc.). Some data may come from medical databases or other database repositories. At 120, once all the data, from all the sources (e.g., 115), is gathered, the example procedure may store the data at 130, in a working database (e.g., 135). Next, at 140, the data may be prepared for modeling.

Fig. 1B illustrates one example procedure for preparing the collected data (e.g., 135). First, at 145, the example procedure may load some or all of the data. At 150, the example procedure may extract features from the data. This may include transforming the data to conform to some standard, mining the data for relevant pieces of information, or otherwise tagging relevant parts of the raw data. Next, at 155, the example procedure may categorize the data. Any variety of categorizations is possible. One example categorization may be diagnoses. For example, at 150, an ICD notation (i.e., "International Classification of Diseases") (e.g., ICD-9) may be pulled from the raw data. Then, at 155, the example procedure may classify this notation according to its position in the ICD code scheme. Other classifications could include procedures, CPT codes (i.e., "Current Procedural Terminology"), or any other category relevant to the modeled outcome. For instance, multiple codes representing related diagnoses may be aggregated to a more general category relevant for all codes to create useful variables for modeling. Next, at 160, the individual records may be aligned according to the time the event occurred. The individual records may also be segmented according to a timeline.

At 165, the records may be aggregated and imported into the modeling algorithm to create one or more models. At 170, outcome variables may be created. In this example embodiment the outcome variable is a hospitalization event within a future timeframe (e.g., a month, week, etc.). Other embodiments for the outcome variable may include the probability of a patient being hospitalized or a score for likelihood of hospitalization which, may be used to rank patients by risk of hospitalization. At 175, the example procedure may create a longitudinal data layout. This data can be used to create time-related variables for individual patient records. An example of this is a variable for "time since last hospitalization". At 180 the data is partitioned to train, test, and validate one or more models. The data may be partitioned so the data which is used to train the model is separate from the data used to test and validate the model. This ensures that the model does not simply learn the training data and can provide good solutions for data it has not been trained on. Validation generally includes multiple models to find one or more with a sufficient level of accuracy. At 185, the example procedure may apply the model to working datasets to predict the probability of the relevant event (e.g., a hospitalization), and/or save the model to a model database (e.g., 195) for future use.

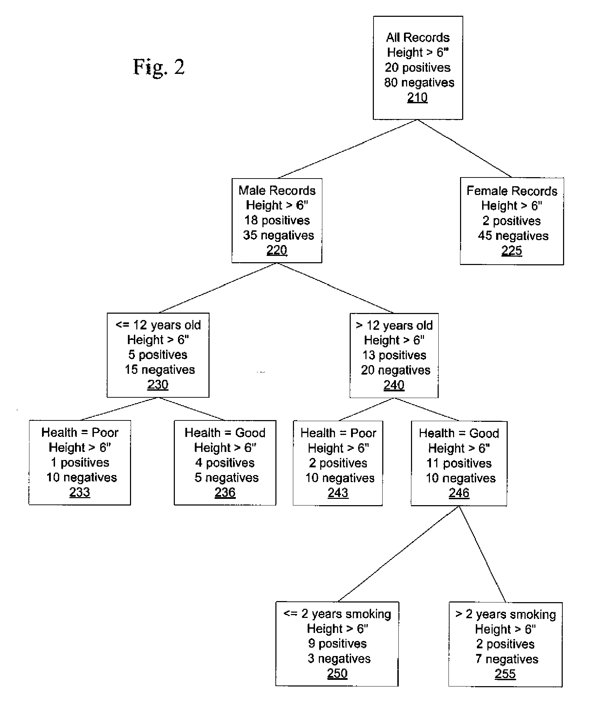

One example method of data partitioning, according to an example embodiment of the present invention, is to train, test, and validate one or more decision trees. Decision trees are formulated by breaking the record data down with the goal of outcome "purity". Outcome purity generally means that data is split based on a criteria, such that the relevant outcome is maximized on one side of the split. In this way, the root of the decision tree may represent the entire data set. The children of the parent (e.g., root) represent record sets split by a criteria (e.g., gender). The goal of this split is to favor leaf nodes (e.g., nodes with no children) with as "pure" an outcome for the relevant criteria as possible. Fig. 2 illustrates an example of this. Root/parent node 210 may represent the entire data set including all the records. In the example illustration of Fig. 2, the relevant criteria is whether or not a person is at least six feet tall or shorter. As root/parent 210 illustrates, the record set has 100 data points (e.g., 100 people), 20 of which satisfy the relevant criteria (e.g., 20 people greater than six feet tall). Next, the decision tree may split (i.e., partition) the record set into child nodes, based on an attribute. The goal is to maximize the quantity of people over six feet tall in one child node, and maximize the quantity of people under six feet tall in another child node. When no further splitting is required of a node, that node will be a leaf node with no children. In the example illustration of Fig. 2, gender is selected as the first relevant attribute to partition on. Child node 220 may now contain all of the records associated with male patients, and child node 225 may now contain all of the records associated with female patients.

If an example partition were to create "pure" leaves, then the records associated with people over 6 feet tall would all fall in one leaf and the records with people under 6 feet tall would all fall in the other leaf. However, though "pure" leaves might not always be possible, Fig. 2 illustrates the desired goal, where each child node is purer than the parent. Parent/root node 210 is 80% under and 20% over (e.g., 80 of 100 records are under 6 feet tall and 20 of 100 records are over). Child node 220 is 34% over, which is a 14 point increase in positive result purity. Child node 225 is 95.7% under, which is a 15.7 point increase in negative result purity. The number of positive outcomes in node 225 is small enough that node 225 may be left as a leaf node, with no further splitting. However, node 220 may be split further to create a higher level of purity in child nodes. For example, nodes 230 and 240 are constructed based on age. Node 230 has all of the males who are 12 years old or younger, which contains 5 people who are at least six feet tall, and 15 who are not. Further, node 240 has all of the males who are older than 12 years old, which contains 13 people who are at least six feet tall, and 20 who are not. Both nodes 230 and 240 may have a sufficient number of positive results to further split into child nodes. At the next level, the nodes are split according to "childhood health". This could be evaluated any number of ways, and may be as simple as asking each participant to rate their childhood health as "good" or "poor". Nodes 233, 236, 243, and 246 show the outcome of this further splitting. The first three of those nodes may remain leaf nodes, and node 246, with the highest number of positive results, may be split further. The final two leaf nodes, 250 and 255, may be created by splitting node 246 based on whether a record indicates more or less than 2 years of adolescent smoking. Node 255, e.g., 12 plus year old males with good childhood health and more than 2 years of smoking as an adolescent, may have 2 positive results (e.g., at least six feet tall) and 7 negative results (e.g., less than six feet tall). Node 250 contains those records that indicate no more than 2 years of adolescent smoking may have 9 positives and 3 negatives.

Additional or alternative splitting may create an even purer concentration. The purity of the leaf nodes may be balanced against the size of the decision tree. For example, it is possible to guaranty completely pure leaf nodes if each leaf node contains only one record. However, a tree may have thousands of records, and single record leaf nodes may require an unreasonable amount of processing overhead to use such a large tree. Therefore, example embodiments of the present invention may balance greater purity against maintaining an efficient tree size. Fig. 2 illustrates a five level tree. However, any number of split criteria could be imposed to create any number of levels to achieve the purest desired concentrations of the relevant outcome in the leaves.

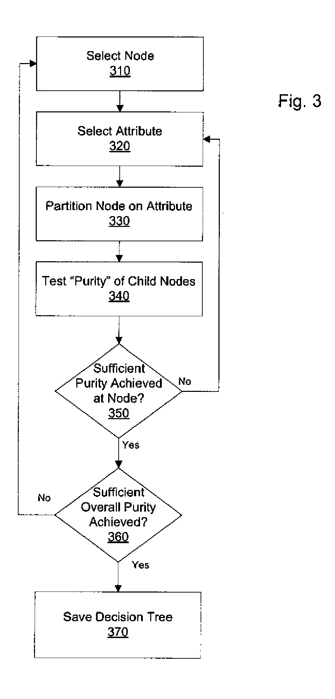

Fig. 3 illustrates one example method of creating a decision tree (e.g., Fig. 2). First, at 310, a node is selected. At the start of the example method, this may be the root node, and may include all of the data records. Next, at 320, an attribute is selected (e.g., gender). The selection may occur at random, may be selected by a person, or may be selected based on some other algorithm or metric. At 330, the node may be partitioned according to the attribute. The partitioning may create two or more child nodes, each with a subset of the data records of the parent node. At 340, the purity of the newly created child nodes may be tested against some configurable threshold. At 350, if sufficient added purity is not achieved for the children of this particular node, then a new attribute may be selected, and the process may be repeated until sufficient added purity is created in the child nodes. Once the child nodes achieve sufficient added purity, the overall purity may be tested against a second configurable threshold. If the overall purity of the decision tree is sufficient, the tree may be saved for model validation at 370. If however, the overall purity is insufficient, then the example procedure may return to 310 and select a new node. The new node may be one of the recently created child nodes, or a sibling node of the node previously partitioned. Fig. 3 is only one example procedure, and many others are possible. For example, example embodiments may save a sufficiently pure tree at 370, and also return to 310 to determine if other variations can create other sufficiently pure decision trees. The other variations could then replace weaker trees, or all sufficient trees may be saved for model verification. Additionally, "sufficiency" does not need to be a configurable threshold, but may be based on any number of things, including "diminishing returns." For example, the example method may execute until the added purity of further iterations is less than some minimal threshold.

Different decision tree algorithms may perform the node partitioning or splitting differently. Additionally, when a tree is constructed, branches that do not meet some minimum threshold of improved purity must be removed (e.g., "pruned" from the tree). Different decision tree algorithms may perform this "pruning" differently. Additionally, it may often be the case that records are missing one or more values. For example, the records associated with a patient may have a large quantity of data, but be missing certain information, even basic information such as gender, age, etc. Different decision tree algorithms may deal with these missing data pieces differently as well. Some algorithms may insert one or more default variables in the missing record, and others may treat the lack of a value as the value (e.g., a binary attribute would have three values, the two known values and "unknown"). The algorithm used to construct the decision tree may depend on the relevant outcome (e.g., a hospitalization event). Chi-squared Automatic Interaction Detector (CHAID) treats missing values as their own value, and is an advantageous algorithm for constructing the decision trees because it includes missing values as legitimate values in the tree splitting process.

One additional problem with creating a model to predict rare events is that the dataset is inherently one-sided. Because the event is "rare" there will be far fewer occurrences of that event than not. However, as with most modeling techniques, a balanced dataset (e.g., one with approximately equal positive and negative relevant outcomes) may create a more accurate model. Data mining models generally need at least semi-balanced datasets to learn how to correctly categorize a positive outcome (e.g., a hospitalization event). Correcting for this disparity usually requires the replication of positive datasets or the elimination of negative datasets. However, example embodiments of the present invention may instead use weighted "misclassification costs." Meaning, a penalty may be assessed when the model incorrectly predicts an outcome. Then, the penalty may be set to achieve an optimized accuracy. For example, if a dataset has 1 positive outcome for every 20 negative outcomes, then the model construction algorithm may assign a 1 point penalty for incorrectly characterizing a negative outcome (e.g., identifying a record set that did not lead to a hospitalization as one that did lead to a hospitalization), and a 20 point penalty for incorrectly characterizing a positive outcome. The mischaracterization cost does not have to be the exact transverse of the outcome proportion. The mischaracterization may likely be inversely proportional to the outcome proportion, but may have a greater or lesser ratio. The ideal ratio of mischaracterization costs may be determined by experimentation and adjustment.

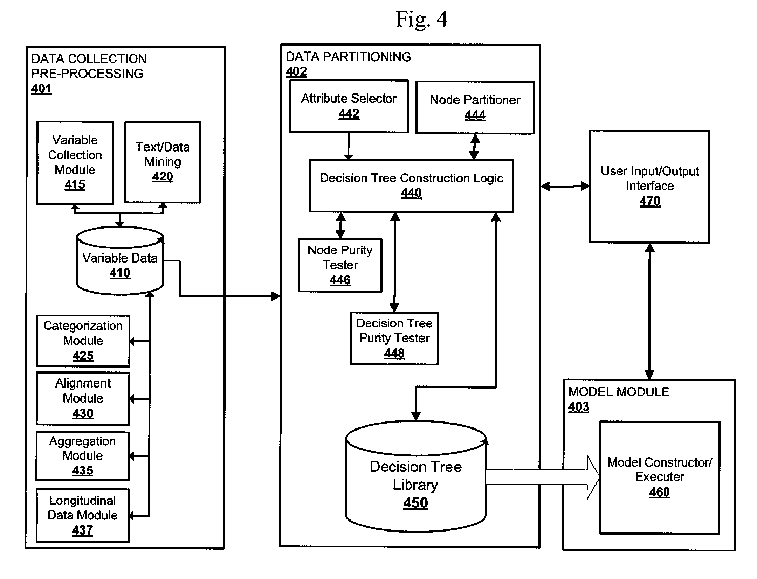

Fig. 4 illustrates an example system according to an example embodiment of the present invention. 401 may illustrate a data collection, preparation, and pre-processing component. This may include a data repository 410 for holding all of the variables used in the model constructing process. There may be a variable collection module 415 that may collect various data records from one or more sources. There may be a text and/or data mining module 420. This module may extract relevant information from textual narratives, journals, diaries, articles, etc. Once these modules (e.g., 415 and 420) collect the relevant data records, other modules may be used to adjust, standardize, and otherwise prepare the data to be organized in a decision tree. For example, a categorization module 425 may organize data according to category, code, relation to other data, or any other relevant criteria. An alignment module 430 may organize the separate data records (each with one or more attributes) to line up based on some dimension (e.g., time). The aggregation module 435 may combine data records and further prepare them for use in the construction of a decision tree. For instance, the same data coming from multiple sources may be received with different characteristics such as name and unit of measure. In addition, different sources may have the same data, but at different levels of detail. For example, one data source may have blood pressure readings for a patient every week whereas another may only have a reading every month. The aggregation module may aggregate like data so that it is mapped to the same variable for modeling with the same baseline characteristics. In addition, the aggregation module may aggregate the data based on the availability of data such as creating a variable for the blood pressure measurements above in monthly buckets since monthly is the most frequently occurring measurement interval. The aggregation module may also aggregate with more complex rules based on the data received and the model being constructed. The longitudinal data module 437 may create a data layout to further prepare the data for use in the construction of a decision tree. This allows variables to be created for each subject which take the longitudinal nature of the data into account. Since patients are measured sequentially over time, the data set-up of the longitudinal data module may allow the creation of variables which exploit the time-relation of measurements within a patient. An example of this may be time since last hospitalization for a patient.

Once the data has been collected, pre-processed, and otherwise prepared for modeling, the variable data may be imported, transmitted, or otherwise made accessible to a data partitioning component 402. This component may be responsible for constructing decision trees for use in the modeling. The component may contain construction logic 440, which may contain a set of rules designed to facilitate the tree construction from the variable data. This component may generally be configured to implement a decision tree construction method, e.g., as illustrated in Fig. 3. There may be an attribute selector 442 to select one or more attributes to base the partitioning on. There may be a node partitioner 444, which may take the selected attribute and create two child nodes connected to the current node being partitioned. Each of these child nodes may have a subset of the records associated with the parent node, based on the value in each record for the selected attribute. Node purity tester 446 may be responsible for determining if a node partition has achieved a minimum level of added purity in the newly created child nodes. Decision tree purity tester 448 may be responsible for determining when a sufficiently pure decision tree is ready to be added to a model, or otherwise used to predict a relevant event. Saved decision trees (e.g., constructed trees passing the decision tree purity tester 448) may be stored in a data repository (e.g., decision tree library 450). The one or more stored decision trees may be sent to a model constructor/executer 460. The decision tree may have been constructed from historical data to create a model capable of predicting some event. The model module 403 may take "live" data, apply the constructed model to the data, and produce an occurrence-probability of the relevant event. There may also be a user I/O interface 470 used to experiment, adjust, and otherwise administrate the example modeling system illustrated in Fig. 4. The example system of Fig. 4 may reside on one or more computer systems. These one or more systems may be connected to a network (e.g., the Internet). The one or more systems may have any number of computer components known in the computer art, such as processors, storage, RAM, cards, input/output devices, etc.

A hospitalization event was used in this description as an example, but is only one example of a rare event that may be predicted by models produced and run by example embodiments of the present invention. Any rare event and data associated with the rare event may be modeled and predicted using example embodiments of the present invention. Example embodiments may predict when a production factory goes offline. Events may include: downtime per each piece of equipment, error messages per each piece of equipment, production output, employee vacations, employee sick days, experience of employees, weather, time of year, power outages, or any number of other metrics related to factory production capacity. Factory data (e.g., records) may be proposed, measured, and assimilated into a model. The model may be used to compare known data about events at a factory. The outcome of that comparison may lead to the probability the factory goes offline. It may be appreciated that any rare event and set of related events may be used in conjunction with example embodiments of the present invention to predict the probability of that rare event occurring.

The various systems described herein may each include a computer-readable storage component for storing machine-readable instructions for performing the various processes as described and illustrated. The storage component may be any type of machine readable medium (i.e., one capable of being read by a machine) such as hard drive memory, flash memory, floppy disk memory, optically-encoded memory (e.g., a compact disk, DVD-ROM, DVD±R, CD-ROM, CD±R, holographic disk), a thermomechanical memory (e.g., scanning-probe-based data-storage), or any type of machine readable (computer readable) storing medium. Each computer system may also include addressable memory (e.g., random access memory, cache memory) to store data and/or sets of instructions that may be included within, or be generated by, the machine-readable instructions when they are executed by a processor on the respective platform. The methods and systems described herein may also be implemented as machine-readable instructions stored on or embodied in any of the above-described storage mechanisms. The various communications and operations described herein may be performed using any encrypted or unencrypted channel, and storage mechanisms described herein may use any storage and/or encryption mechanism.

Aspects and embodiments of the present invention include:
(a) A method, comprising:
   loading a plurality of data records, wherein each data record has one or more attributes, wherein the plurality of data records include a first group;
   assigning a relevant event to be predicted;
   selecting at least one of the one or more attributes;
   creating a plurality of subgroups associated with the first group, wherein each data record associated with the first group is associated with at least one subgroup, wherein the associating for each record is based at least in part on a respective value associated with the selected attribute; and
   repeating the selecting and creating until a concentration of positive outcomes for the relevant event is sufficient.

Sufficient preferably includes a user defined threshold.

The repeating preferably includes measuring a difference between a concentration attained before the repeating and a concentration attained after the repeating, and wherein sufficient preferably includes the difference being below a threshold.

The first group is preferably a root node of a decision tree and the plurality of subgroups are preferably child nodes of the decision tree. The decision tree is preferably a binary tree.

The relevant event is preferably a hospitalization event within a timeframe.

The plurality of data records preferably includes health related records.

The method preferably further comprises using at least the first group and the associated plurality of subgroups to predict a probability of the relevant event occurring within a timeframe. The relevant event is preferably associated with an entity, and wherein the using preferably includes applying the first group and the associated plurality of subgroups to a dataset, wherein the dataset is preferably associated with the entity.
(b) A system, comprising:
   a memory configured to load a plurality of data records, wherein each data record has one or more attributes, wherein the plurality of data records include a first group;
   a processor configured to assign a relevant event to be predicted;
   the processor configured to select at least one of the one or more attributes;
   the processor configured to create a plurality of subgroups associated with the first group, wherein each data record associated with the first group is associated with at least one subgroup, wherein the associating for each record is based at least in part on a respective value associated with the selected attribute;
   the processor further configured to repeat the selecting and creating until a concentration of positive outcomes for the relevant event is sufficient.

Sufficient preferbaly includes a user defined threshold.

The repeating preferably includes measuring a difference between a concentration attained before the repeating and a concentration attained after the repeating, and wherein sufficient preferably includes the difference being below a threshold.

The first group is preferably a root node of a decision tree and the plurality of subgroups are preferably child nodes of the decision tree. The decision tree is preferably a binary tree.

The relevant event is preferably a hospitalization event within a timeframe.

The plurality of data records preferably includes health related records.

The system preferably further comprises the processor configured to predict a probability of the relevant event occurring within a timeframe using at least the first group and the associated plurality of subgroups. The relevant event is preferably associated with an entity, and wherein the using preferably includes applying the first group and the associated plurality of subgroups to a dataset, wherein the dataset is preferably associated with the entity.
(c) A computer-readable storage medium encoded with instructions configured to be executed by a processor, the instructions which, when executed by the processor, cause the performance of a method, comprising:
   loading a plurality of data records, wherein each data record has one or more attributes, wherein the plurality of data records include a first group;
   assigning a relevant event to be predicted;
   selecting at least one of the one or more attributes;
   creating a plurality of subgroups associated with the first group, wherein each data record associated with the first group is associated with at least one subgroup, wherein the associating for each record is based at least in part on a respective value associated with the selected attribute; and
   repeating the selecting and creating until a concentration of positive outcomes for the relevant event is sufficient.

Although the present invention has been described with reference to particular examples and embodiments, it is understood that the present invention is not limited to those examples and embodiments. The present invention as claimed therefore includes variations from the specific examples and embodiments described herein, as will be apparent to one of skill in the art.

## Claims

1. A method, comprising:
loading a plurality of data records, wherein each data record has one or more attributes, wherein the plurality of data records include a first group;
assigning a relevant event to be predicted;
selecting at least one of the one or more attributes;
creating a plurality of subgroups associated with the first group, wherein each data record associated with the first group is associated with at least one subgroup, wherein the associating for each record is based at least in part on a respective value associated with the selected attribute; and
repeating the selecting and creating until a concentration of positive outcomes for the relevant event is sufficient.

2. A method as claimed in claim 1, further comprising:
using at least the first group and the associated plurality of subgroups to predict a probability of the relevant event occurring within a timeframe.

3. A method as claimed in claim 2, wherein the relevant event is associated with an entity, and wherein the using includes applying the first group and the associated plurality of subgroups to a dataset, wherein the dataset is associated with the entity.

4. A system, comprising:
a memory configured to load a plurality of data records, wherein each data record has one or more attributes, wherein the plurality of data records include a first group;
a processor configured to assign a relevant event to be predicted;
the processor configured to select at least one of the one or more attributes;
the processor configured to create a plurality of subgroups associated with the first group, wherein each data record associated with the first group is associated with at least one subgroup, wherein the associating for each record is based at least in part on a respective value associated with the selected attribute;
the processor further configured to repeat the selecting and creating until a concentration of positive outcomes for the relevant event is sufficient.

5. A method as claimed in any of claims 1 to 3 or a system as claimed in claim 4, wherein sufficient includes a user defined threshold.

6. A method as claimed in any of claims 1 to 3 and 5 or a system as claimed in claim 4 or claim 5, wherein the repeating includes measuring a difference between a concentration attained before the repeating and a concentration attained after the repeating, and wherein sufficient includes the difference being below a threshold.

7. A method as claimed in any of claims 1 to 3, 5 and 6 or a system as claimed in any of claims 4 to 6, wherein the first group is a root node of a decision tree and the plurality of subgroups are child nodes of the decision tree.

8. A method as claimed in any of claims 1 to 3 and 5 to 7 or a system as claimed in claim 7, wherein the decision tree is a binary tree.

9. A method as claimed in any of claims 1 to 3 and 5 to 8 or a system as claimed in any of claims 4 to 8, wherein the relevant event is a hospitalization event within a timeframe.

10. A method as claimed in any of claims 1 to 3 and 5 to 9 or a system as claimed in any of claims 4 to 9, wherein the plurality of data records includes health related records.

11. A system as claimed in any of claims 4 to 10, further comprising:
the processor configured to predict a probability of the relevant event occurring within a timeframe using at least the first group and the associated plurality of subgroups.

12. A system as claimed in claim 11, wherein the relevant event is associated with an entity, and wherein the using includes applying the first group and the associated plurality of subgroups to a dataset, wherein the dataset is associated with the entity.

13. A computer-readable storage medium encoded with instructions configured to be executed by a processor, the instructions which, when executed by the processor, cause the performance of a method, comprising:
loading a plurality of data records, wherein each data record has one or more attributes, wherein the plurality of data records include a first group;
assigning a relevant event to be predicted;
selecting at least one of the one or more attributes;
creating a plurality of subgroups associated with the first group, wherein each data record associated with the first group is associated with at least one subgroup, wherein the associating for each record is based at least in part on a respective value associated with the selected attribute; and
repeating the selecting and creating until a concentration of positive outcomes for the relevant event is sufficient.
